# EUROPEAN PATENT APPLICATION

(11) **EP 4 831 152 A1**
(43) Date of publication of application: **28.10.2026**
(21) Application number: 26172987.5
(22) Date of filing: 17.04.2026
(51) Int. Cl.: C07C 31/125

(54) **2-HALO-6,10-DIMETHYLTRIDECANE COMPOUND, PROCESS FOR PREPARING THE SAME, AND PROCESS FOR PREPARING ALKYL 2,6,10-TRIMETHYLTRIDECANOATE COMPOUND THEREFROM**

(30) Priority: 24.04.2025 JP 2025072457
(71) Applicant: SHIN-ETSU CHEMICAL CO., LTD., Tokyo 100-0005 (JP)
(72) Inventor: MIYAKE, Yuki, Niigata, 942-8601 (JP); WATANABE, Takeru, Niigata, 942-8601 (JP)
(74) Representative: De Vries & Metman

(57) **Abstract**

The present invention relates to a 2-halo-6,10-dimethyltridecane compound (1), wherein X¹ represents a halogen atom. The present invention further relates to process for preparing the aforesaid 2-halo-6,10-dimethyltridecane compound (1), the process comprising steps of subjecting a nucleophilic reagent, 3,7-dimethyldecyl compound (2), wherein M¹ represents Li, MgZ¹, CuZ¹, or CuLiZ¹, and Z¹ represents a halogen atom or a 3,7-dimethyldecyl group, to a nucleophilic addition reaction with propylene oxide (3) to form 6,10-dimethyl-2-tridecanol (4), and reacting the 6,10-dimethyl-2-tridecanol (4) thus obtained with a halogenating agent (5) to form the aforesaid 2-halo-6,10-dimethyltridecane compound (1).

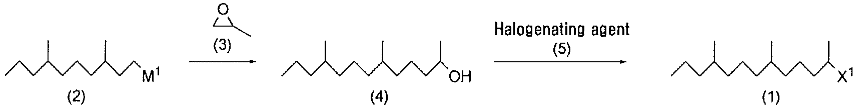

## Description

### TECHNICAL FIELD

An embodiment of the present invention relates to a 2-halo-6,10-dimethyltridecane compound, a process for preparing a 2-halo-6,10-dimethyltridecane compound, and a process for preparing an alkyl 2,6,10-trimethyltridecanoate compound from the 2-halo-6,10-dimethyltridecane compound.

### BACKGROUND ART

The Neotropical brown stink bug (scientific name: *Euschistus heros*) is a South American soybean pest that feeds on stems, leaves, pods, and seeds of soybeans. It is known that *Euschistus heros* larvae of two years or more damage seeds, and the extent of the damage becomes more severe as developmental stages proceed (Non-Patent Literature 1 listed below). Control is therefore especially important in the early stages of development, and control with pesticides (insecticides) is currently in widespread use. Control with typical pesticides, however, is difficult because it is often ineffective. Meanwhile, concerns regarding residual pesticides and the development of resistance have focused attention on biological control methods, with high expectations for the use of pheromones (Non-Patent Literature 2 listed below).

The pheromone composition of *Euschistus heros* is reported to be a mixture of an alkyl 2,6,10-trimethyltridecanoate compound, 2,6,10-trimethyltridecanoic acid methyl ester (methyl 2,6,10-trimethyltridecanoate), as well as 2,6,10-trimethyldodecanoic acid methyl ester (methyl 2,6,10-trimethyldodecanoate) and methyl (2E,4E)-2,4-decadienoate (Non-Patent Literatures 3 and 4 listed below).

It is reported that the major component, methyl 2,6,10-trimethyltridecanoate, of this mixture may be prepared by the following steps. For example, propylmagnesium bromide is subjected to an addition reaction with cyclopropyl methyl ketone in a diethyl ether solvent to form 2-cyclopropyl-2-pentanol. The 2-cyclopropyl-2-pentanol thus obtained is then reacted with 48% hydrobromic acid to form 1-bromo-4-methyl-3-heptene. The 1-bromo-4-methyl-3-heptene thus obtained is then converted into a Grignard reagent, followed by subjecting the Grignard reagent to an addition reaction with cyclopropyl methyl ketone to form 2-cyclopropyl-6-methyl-5-nonen-2-ol. The 2-cyclopropyl-6-methyl-5-nonen-2-ol thus obtained is then reacted with 48% hydrobromic acid to form 1-bromo-4,8-dimethyl-3,7-undecadiene. Next, dimethyl 2-methylmalonate is deprotonated with sodium hydride, and then subjected to a malonic acid ester synthesis reaction with the aforesaid 1-bromo-4,8-dimethyl-3,7-undecadiene thus obtained to form dimethyl 2-methyl-2-(4,8-dimethyl-3,7-undecadienyl)malonate. The dimethyl 2-methyl-2-(4,8-dimethyl-3,7-undecadienyl)malonate thus obtained is subjected to Krapcho decarboxylation with sodium chloride and water in a dimethyl sulfoxide (DMSO) solvent to form methyl 2,6,10-trimethyl-5,9-tridecadienoate. The methyl 2,6,10-trimethyl-5,9-tridecadienoate thus obtained is then subjected to a hydrogenation reaction in the presence of a palladium-carbon (Pd/C) catalyst to obtain methyl 2,6,10-trimethyltridecanoate (Non-Patent Literature 5 listed below).

It is also reported that methyl 2,6,10-trimethyltridecanoate may be prepared by another process having the following steps. For example, 2-methylpentanol is brominated with bromine and triphenylphosphine to form 1-bromo-2-methylpentane. Also, (±)-citronellol is tosylated with *p*-toluenesulfonyl chloride in a pyridine solution to form citronellyl tosylate. The aforesaid 1-bromo-2-methylpentane thus obtained is reacted with magnesium to form a Grignard reagent, followed by subjecting the Grignard reagent to a coupling reaction with the aforesaid citronellyl tosylate in the presence of a copper catalyst to form 2,6,10-trimethyl-2-tridecene. The 2,6,10-trimethyl-2-tridecene thus obtained is subjected to an oxidation reaction with selenium oxide in *tert-*butyl hydroperoxide, and then subjected to a reduction reaction with sodium borohydride to form 2,6,10-trimethyl-2-tridecen-1-ol. The 2,6,10-trimethyl-2-tridecen-1-ol thus obtained is then subjected to a hydrogenation reaction in the presence of platinum oxide (an Adams catalyst) to form 2,6,10-trimethyl-1-tridecanol. The 2,6,10-trimethyl-1-tridecanol thus obtained is then subjected to Jones oxidation with chromic acid followed by methylating carboxylic acid with diazomethane to form methyl 2,6,10-trimethyltridecanoate (Non-Patent Literature 6 listed below).

### PRIOR ART

### [Non-Patent Literatures]

[Non-Patent Literature 1] Magda Andreia Tessmerl et al., Neotropical Entomology., 2022, 51, 112-121.
[Non-Patent Literature 2] Elisabeth A. Hodgdon et al., The Canadian Entomologist, 2022, 154, e37, 1-17.
[Non-Patent Literature 3] M. Borges et al., J, Appl. Ent., 1998, 122, 335-338.
[Non-Patent Literature 4] Aijun Zhang et al., Neotropical Entomology, 2003, 32 (4), 713-717.
[Non-Patent Literature 5] Kenji Mori et al., Liebigs Ann. Chem., 1994, 637-639.
[Non-Patent Literature 6] Paulo H. G. Zarbin et al., J. Chem. Ecol., 2000, 26 (12), 2737-2746.

### OBJECT OF THE INVENTION

However, the process for preparing the methyl 2,6,10-trimethyltridecanoate of Non-Patent Literature 5 is unsuited to industrialization due to the use of sodium hydride and palladium-carbon, which are highly ignitable. The preparation process also is unsuited to industrialization because dimethyl sulfoxide, which is used in a large amount as a solvent, requires special equipment, is intensely malodorous, and is drawing concern as a possible reproductive toxin. Furthermore, the process for preparing methyl 2,6,10-trimethyltridecanoate of Non-Patent Literature 5 is not economical due to the use of the expensive raw material, cyclopropyl methyl ketone, and the expensive reagent, palladium-carbon. The recent dramatic price increase of palladium makes inexpensive preparation especially difficult. Additionally, the process for preparing methyl 2,6,10-trimethyltridecanoate of Non-Patent Literature 5 is a long process with a total of seven steps and a medium seven-step yield of 30.34%.

Meanwhile, the process for preparing methyl 2,6,10-trimethyltridecanoate of Non-Patent Literature 6 uses selenium oxide and chromic acid, which have extremely high environmental toxicity, and are undesirable in view of green chemistry. Moreover, industrialization is difficult due to the use of *tert*-butyl hydroperoxide, which is explosive in high purity. The use of expensive platinum oxide is not economical. Furthermore, the process for preparing methyl 2,6,10-trimethyltridecanoate of Non-Patent Literature 6 is a long process with a total of six steps and a low six-step yield of 14.47%.

The preparation processes of both Non-Patent Literatures 5 and 6 purify methyl 2,6,10-trimethyltridecanoate by silica gel column chromatography, making the processes unsuited to industrial applications because purification by silica gel column chromatography is difficult to carry out on an industrial scale of 100 kg or more.

The present invention has been made in view of the aforementioned circumstances, and aims to provide a novel compound that is a synthetic intermediate for efficiently preparing an alkyl 2,6,10-trimethyltridecanoate compound. The present invention also aims to provide a process for preparing the novel compound, and a process for preparing an alkyl 2,6,10-trimethyltridecanoate compound from the novel compound.

### SUMMARY OF THE INVENTION

As a result of intensive research to overcome the aforesaid problems of the prior art, the present inventors found the novel compound, 2-halo-6,10-dimethyltridecane compound. The 2-halo-6,10-dimethyltridecane compound according to an embodiment of the present invention may be prepared inexpensively and in large amounts, and may be purified with only distillation. Furthermore, alkyl 2,6,10-trimethyltridecanoate compounds including the pheromone of *Euschistus heros*, methyl 2,6,10-trimethyltridecanoate, may be comprehensively and efficiently synthesized by individually making various alkyl 2,6,10-trimethyltridecanoate compounds in the final step.

According to a first aspect of the present invention, there is provided a 2-halo-6,10-dimethyltridecane compound of the following general formula (1): 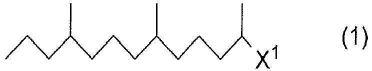 wherein X¹ represents a halogen atom.

According to a second aspect of the present invention, there is provided a process for preparing a 2-halo-6,10-dimethyltridecane compound of the following general formula (1): 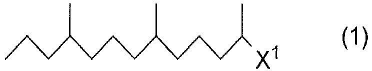
wherein X¹ represents a halogen atom,
the process comprising steps of:
   subjecting a nucleophilic reagent, 3,7-dimethyldecyl compound, of the following general formula (2): 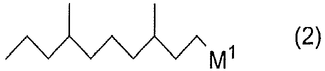
      wherein M¹ represents Li, MgZ¹, CuZ¹, or CuLiZ¹, and Z¹ represents a halogen atom or a 3,7-dimethyldecyl group,
      to a nucleophilic addition reaction with propylene oxide of the following formula (3): 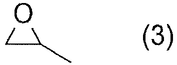
      to form 6,10-dimethyl-2-tridecanol of the following formula (4): 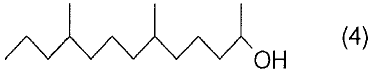 and
   reacting the 6,10-dimethyl-2-tridecanol (4) thus obtained with a halogenating agent (5) to form the aforesaid 2-halo-6,10-dimethyltridecane compound (1).

According to a third aspect of the present invention, there is provided the aforesaid process for preparing the 2-halo-6,10-dimethyltridecane compound (1), the process further comprising steps of:
subjecting a nucleophilic reagent, 2-methylpentyl compound, of the following general formula (6): 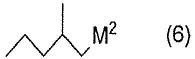
   wherein M² represents Li, MgZ², CuZ², or CuLiZ², and Z² represents a halogen atom or a 2-methylpentyl group,
   to a coupling reaction with a sulfonate compound (7) of the following general formula (7): 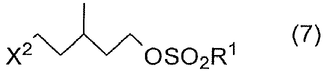
   wherein X² represents a halogen atom, and R¹ represents a hydrocarbon group having 1 to 7 carbon atoms,
   to form a 1-halo-3,7-dimethyldecane compound of the following general formula (8): 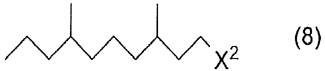
   wherein X² represents a halogen atom,
   and
converting the 1-halo-3,7-dimethyldecane compound (8) thus obtained into the aforesaid nucleophilic reagent, 3,7-dimethyldecyl compound (2).

According to a fourth aspect of the present invention, there is provided a process for preparing an alkyl 2,6,10-trimethyltridecanoate compound of the following general formula (11): 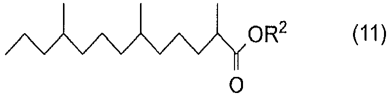
wherein R² represents an alkyl group having 1 to 12 carbon atoms,
the process comprising steps of:
   converting a 2-halo-6,10-dimethyltridecane compound of the following general formula (1): 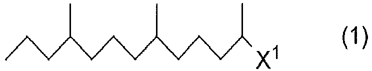
      wherein X¹ represents a halogen atom,
      into a nucleophilic reagent, 1,5,9-trimethyldodecyl compound, of the following general formula (9): 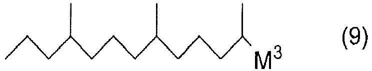 and
   subjecting the nucleophilic reagent, 1,5,9-trimethyldodecyl compound (9) thus obtained, to a nucleophilic substitution reaction with alkyl haloformate of the following general formula (10): 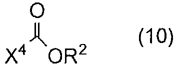
wherein X⁴ represents a halogen atom, and R² represents an alkyl group having 1 to 12 carbon atoms,
to form the aforesaid alkyl 2,6,10-trimethyltridecanoate compound (11).

According to an embodiment of the present invention, the target compound, alkyl 2,6,10-trimethyltridecanoate compound, may be prepared efficiently with a low environmental impact, in a high yield, and in fewer steps. According to an embodiment of the present invention, the target compound, alkyl 2,6,10-trimethyltridecanoate compound, may be economically prepared. According to an embodiment of the present invention, the steps produce substantially no compounds similar to the target compound. Purification by column chromatography is therefore unnecessary, making possible purification by distillation, which may be scaled up. According to an embodiment of the present invention, there is provided a novel synthetic intermediate useful for preparing the aforesaid alkyl 2,6,10-trimethyltridecanoate compound.

### DETAILED DESCRIPTION OF THE INVENTION

### (I) Process for preparing 2-halo-6,10-dimethyltridecane compound of the following general formula (1)

### (i) A 2-halo-6,10-dimethyltridecane compound (1) is represented by the following general formula (1):

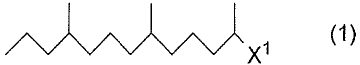

### (ii) A process for preparing a 2-halo-6,10-dimethyltridecane compound (1) will be explained in detail below.

A 2-halo-6,10-dimethyltridecane compound (1) may be prepared, for example, as shown by the following chemical reaction formula, by converting a 1-halo-2-methylpentane compound of the following general formula (12) into a nucleophilic reagent, 2-methylpentyl compound, of the following general formula (6), then subjecting the nucleophilic reagent, 2-methylpentyl compound (6), to a coupling reaction with a sulfonate compound of the following general formula (7) to form a 1-halo-3,7-dimethyldecane compound (8) of the following general formula (8), then converting the aforesaid 1-halo-3,7-dimethyldecane compound (8) into a nucleophilic reagent, 3,7-dimethyldecyl compound, of the following general formula (2), followed by subjecting the nucleophilic reagent, 3,7-dimethyldecyl compound (2), to a nucleophilic addition reaction with propylene oxide of the following general formula (3) to form 6,10-dimethyl-2-tridecanol of the following general formula (4), and then reacting the aforesaid 6,10-dimethyl-2-tridecanol (4) with a halogenating agent (5).

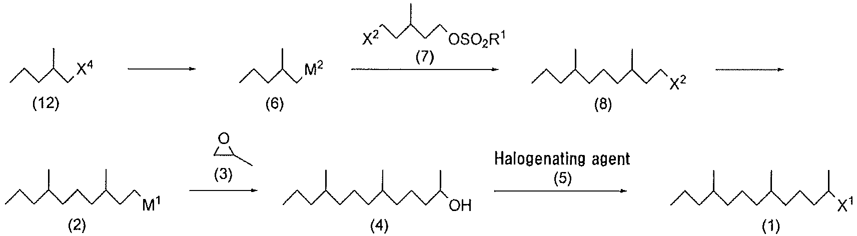

(a) First, a 2-halo-6,10-dimethyltridecane compound (1) and a process for preparing a 2-halo-6,10-dimethyltridecane compound (1) will be explained.

A 2-halo-6,10-dimethyltridecane compound (1) is represented by the following general formula (1):

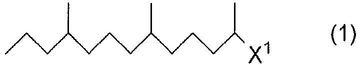

In the general formula (1) above, X¹ represents a halogen atom. Specific examples of the halogen atom X¹ include a chlorine atom, a bromine atom, and an iodine atom. The halogen atom X¹ is preferably a chlorine atom and a bromine atom. By using said chlorine atom and bromine atom, a preferred reactivity may be ensured. The halogen atom X¹ is more preferably a chlorine atom. By using said chlorine atom, a more preferred reactivity may be ensured.

Specific examples of the 2-halo-6,10-dimethyltridecane compound (1) include 2-chloro-6,10-dimethyltridecane, 2-bromo-6,10-dimethyltridecane, and 2-iodo-6,10-dimethyltridecane. The 2-halo-6,10-dimethyltridecane compound (1) is preferably 2-chloro-6,10-dimethyltridecane and 2-bromo-6,10-dimethyltridecane. By using said 2-chloro-6,10-dimethyltridecane and 2-bromo-6,10-dimethyltridecane, preferred storage stability may be ensured. The 2-halo-6,10-dimethyltridecane compound (1) is more preferably 2-chloro-6,10-dimethyltridecane. By using said 2-chloro-6,10-dimethyltridecane, more preferred storage stability may be ensured.

(b) A halogenation reaction of 6,10-dimethyl-2-tridecanol (4) and a halogenating agent (5) will be explained in detail below.

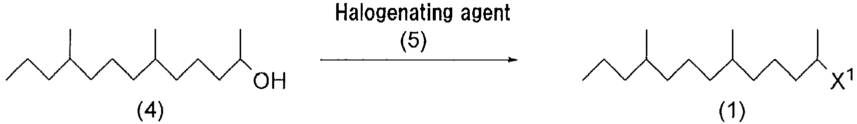

The halogenation reaction may be carried out, for example, by tosylating the hydroxy group of 6,10-dimethyl-2-tridecanol (4) with a *p*-toluenesulfonyl halide compound, and then halogenating the hydroxy group of 6,10-dimethyl-2-tridecanol (4) with a metal salt, lithium halide compound, or by directly halogenating the hydroxy group of 6,10-dimethyl-2-tridecanol (4) with a halogenating agent.

Examples of the halogenating agent (5) include halogen molecules such as chlorine, bromine, and iodine; hydrogen halide compounds such as hydrogen chloride, hydrogen bromide, and hydrogen iodide; methanesulfonyl halide compounds such as methanesulfonyl chloride, methanesulfonyl bromide, and methanesulfonyl iodide; benzenesulfonyl halide compounds such as benzenesulfonyl chloride, benzenesulfonyl bromide, and benzenesulfonyl iodide; *p*-toluenesulfonyl halide compounds such as *p-*toluenesulfonyl chloride, *p*-toluenesulfonyl bromide, and *p*-toluenesulfonyl iodide; thionyl halide compounds such as thionyl chloride, thionyl bromide, and thionyl iodide; phosphorus halide compounds such as phosphorus trichloride, phosphorus pentachloride, and phosphorus tribromide; carbon tetrahalide compounds such as carbon tetrachloride, carbon tetrabromide, and carbon tetraiodide; alkylsilyl halide compounds such as trimethylsilyl chloride, trimethylsilyl bromide, trimethylsilyl iodide, triethylsilyl chloride, triethylsilyl bromide, triethylsilyl iodide, triisopropylsilyl chloride, triisopropylsilyl bromide, triisopropylsilyl iodide, *tert*-butyldimethylsilyl chloride, *tert*-butyldimethylsilyl bromide, and *tert*-butyldimethylsilyl iodide; oxalyl halide compounds such as oxalyl chloride, oxalyl bromide, and oxalyl iodide; and *N*-halosuccinimide compounds such as *N*-chlorosuccinimide, *N*-bromosuccinimide, and *N*-iodosuccinimide. The halogenating agent (5) is preferably methanesulfonyl halide compounds, benzenesulfonyl halide compounds, *p*-toluenesulfonyl halide compounds, and thionyl halide compounds. By using said methanesulfonyl halide compounds, benzenesulfonyl halide compounds, *p-*toluenesulfonyl halide compounds, and thionyl halide compounds, a preferred suppression of side reactions may be ensured. The halogenating agent (5) is more preferably benzenesulfonyl halide compounds and *p*-toluenesulfonyl halide compounds. By using said benzenesulfonyl halide compounds and *p*-toluenesulfonyl halide compounds, a more preferred suppression of side reactions and offensive odors may be ensured. The halogenating agent (5) is even more preferably benzenesulfonyl halide compounds. By using said benzenesulfonyl halide compounds, an even more preferred suppression of side reactions and offensive odors may be ensured.

The halogenating agent may be used alone or in combination thereof, if necessary. The halogenating agent may be a commercially available one.

The amount of the halogenating agent used, per mol of 6,10-dimethyl-2-tridecanol (4), is preferably 0.8 to 5.0 mol, and more preferably 1.0 to 2.5 mol. By using said preferred amount and said more preferred amount, a preferred reactivity and a more preferred reactivity may be ensured.

A base may be incorporated in the halogenation reaction, if necessary.

Examples of the base include hydroxides such as sodium hydroxide, potassium hydroxide, calcium hydroxide, and magnesium hydroxide; carbonates such as sodium carbonate, potassium carbonate, calcium carbonate, and magnesium carbonate; and amines such as triethylamine, *N*,*N*-diisopropylethylamine, piperidine, pyrrolidine, pyridine, lutidine, 4-dimethylaminopyridine, *N*,*N*-dimethylaniline, *N*,*N*-diethylaniline, and 1,8-diazabicyclo[5.4.0]-7-undecene (DBU).

When a methanesulfonyl halide compound, a benzenesulfonyl halide compound, or a *p*-toluenesulfonyl halide compound is used as the aforesaid halogenating agent, the base is preferably amines, and more preferably pyridines such as pyridine, lutidine, and 4-dimethylaminopyridine.

When a thionyl halide compound is used as the halogenating agent, the base is preferably amines, and more preferably trialkylamines such as triethylamine.

The base may be used alone or in combination thereof, if necessary. The base may be a commercially available one.

When the base is used, the amount of the base used, per mol of 6,10-dimethyl-2-tridecanol (4), is preferably more than 0 up to 8.0 mol, and more preferably more than 0 up to 3.0 mol. By using said preferred amount and said more preferred amount, a preferred yield and/or economy and a more preferred yield and/or economy may be ensured.

A metal salt may be incorporated in the halogenation reaction, if necessary.

Examples of the metal salt include lithium salts such as lithium chloride, lithium bromide, and lithium iodide; sodium salts such as sodium chloride, sodium bromide, and sodium iodide; potassium salts such as potassium chloride, potassium bromide, and potassium iodide; calcium salts such as calcium chloride, calcium bromide, and calcium iodide; and magnesium salts such as magnesium chloride, magnesium bromide, and magnesium iodide.

When, for example, tosylating and then halogenating with the metal salt, lithium halide compound, the reaction is carried out with lithium salts such as lithium chloride, lithium bromide, and lithium iodide.

The metal salt may be used alone or in combination thereof, if necessary. The metal salt may be a commercially available one.

When the metal salt is used, the amount of the metal salt used, per mol of 6,10-dimethyl-2-tridecanol (4), is preferably more than 0 up to 30.0 mol, and more preferably more than 0 up to 5.0 mol. By using said preferred amount and said more preferred amount, a preferred reactivity and a more preferred reactivity may be ensured.

Although the metal salt increases the concentration of halide ions in-situ to thereby enhance the reactivity, it is preferred not to incorporate the metal salt in the reaction. By not incorporating the metal salt, preferred economy and/or environmental protection may be ensured.

A solvent may be incorporated in the halogenation reaction, if necessary. Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; polar solvents such as *N*,*N*-dimethylformamide (DMF), *N*,*N*-dimethylacetamide (DMAC), *N*-methylpyrrolidone (NMP), dimethyl sulfoxide (DMSO), γ-butyrolactone (GBL), acetonitrile, acetone, *N*,*N'*-dimethylpropylene urea (DMPU), hexamethylphosphoric triamide (HMPA), dichloromethane, and chloroform; and ester solvents such as methyl acetate, ethyl acetate, n-propyl acetate, and n-butyl acetate. The solvent is preferably 2-methyltetrahydrofuran, 4-methyltetrahydropyran, dichloromethane, chloroform, γ-butyrolactone, *N*-methylpyrrolidone, *N*,*N-*dimethylformamide, *N*,*N*-dimethylacetamide, and acetonitrile. By using said 2-methyltetrahydrofuran, 4-methyltetrahydropyran, dichloromethane, chloroform, γ-butyrolactone, *N-*methylpyrrolidone, *N*,*N-*dimethylformamide, *N*,*N*-dimethylacetamide, and acetonitrile, a preferred reactivity may be ensured. The solvent is more preferably 2-methyltetrahydrofuran, γ-butyrolactone, and acetonitrile. By using said 2-methyltetrahydrofuran, γ-butyrolactone, and acetonitrile, more preferred safety may be ensured. The solvent is even more preferably γ-butyrolactone. By using said γ-butyrolactone, even more preferred safety may be ensured.

The solvent may be used alone or in combination thereof, if necessary. The solvent may be a commercially available one.

When a solvent is incorporated in the halogenation reaction, the amount of the solvent used, per mol of 6,10-dimethyl-2-tridecanol (4), is preferably more than 0 up to 3,000 g, more preferably more than 0 up to 1,500 g, even more preferably more than 0 up to 800 g, and particularly preferably 200 to 600 g.

When the solvent is incorporated, the quantity of starting materials and ease of preparation are reduced, and so the reaction may be carried out without the aforesaid solvent by using a base as the solvent.

The reaction temperature of the halogenation reaction varies, depending on the halogenating agent used, and is preferably -20 to 180°C, more preferably -10 to 120°C, and even more preferably 0 to 70°C. By using said preferred reaction temperature, said more preferred reaction temperature, and said even more preferred reaction temperature, a preferred reactivity, a more preferred reactivity, and an even more preferred reactivity may be ensured.

The reaction time of the halogenation reaction varies, depending on the halogenating agent used and/or the production scale, and is preferably 0.5 to 100 hours. By using said preferred reaction time, a preferred reactivity may be ensured.

Examples of the purification method of the halogenation reaction include purification by silica gel column chromatography and purification by distillation. The aforesaid purification method is preferably purification by distillation. By using said purification by distillation, a preferred industrial production may be ensured.

### (II) 6,10-Dimethyl-2-tridecanol of the following formula (4), and process for preparing 6,10-dimethyl-2-tridecanol (4)

### (i) 6,10-Dimethyl-2-tridecanol (4) is represented by the following formula (4):

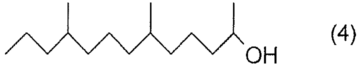

### (ii) A process for preparing 6,10-dimethyl-2-tridecanol (4) will be explained in detail below.

6,10-Dimethyl-2-tridecanol (4) may be prepared, for example, as shown by the following chemical reaction formula, by converting a 1-halo-3,7-dimethyldecane compound (8) into a nucleophilic reagent, 3,7-dimethyldecyl compound (2), and then subjecting the nucleophilic reagent, 3,7-dimethyldecyl compound (2), to a nucleophilic addition reaction with propylene oxide (3).

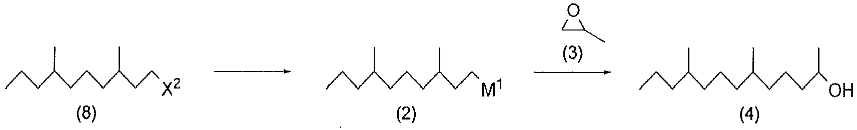

(a) First, the aforesaid nucleophilic reagent, 3,7-dimethyldecyl compound (2), will be explained in detail below. The nucleophilic reagent, 3,7-dimethyldecyl compound (2), is represented by the following general formula (2):

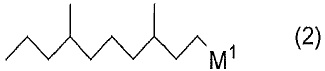

In the general formula (2) above, M¹ represents Li, MgZ¹, CuZ¹, or CuLiZ¹, and Z¹ represents a halogen atom or a 3,7-dimethyldecyl group. Specific examples of the halogen atom Z¹ include a chlorine atom, a bromine atom, and an iodine atom. The halogen atom Z¹ is preferably a chlorine atom and a bromine atom. By using said chlorine atom and bromine atom, a preferred reactivity may be ensured. The halogen atom Z¹ is more preferably a chlorine atom. By using said chlorine atom, a more preferred reactivity may be ensured.

Specific examples of the nucleophilic reagent, 3,7-dimethyldecyl compound (2), include the following compounds:
3,7-dimethyldecyllithium (when M¹ = Li);
3,7-dimethyldecylmagnesium halide compounds (when M¹ = MgZ¹) such as 3,7-dimethyldecylmagnesium chloride, 3,7-dimethyldecylmagnesium bromide, and 3,7-dimethyldecylmagnesium iodide (i.e., Grignard reagents);
bis(3,7-dimethyldecyl)cuprate compounds (when M¹ = CuZ¹) such as bis(3,7-dimethyldecyl)cuprate; and
Gilman reagents (when M¹ = CuLiZ¹) such as lithium bis(3,7-dimethyldecyl)cuprate compounds such as lithium bis(3,7-dimethyldecyl)cuprate (i.e., Gilman reagents).
Grignard reagents such as 3,7-dimethyldecylmagnesium halide compounds are preferred. By using said Grignard reagents such as 3,7-dimethyldecylmagnesium halide compounds, a preferred reactivity may be ensured.

(b) A process for preparing the aforesaid nucleophilic reagent, 3,7-dimethyldecyl compound (2), will be explained in detail below.

The nucleophilic reagent, 3,7-dimethyldecyl compound (2), may be prepared from a 1-halo-3,7-dimethyldecane compound (8) below.

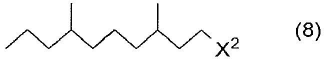

In the general formula (8) above, X² represents a halogen atom. Specific examples of the halogen atom X² include a chlorine atom, a bromine atom, and an iodine atom. The halogen atom X² is preferably a chlorine atom and a bromine atom. By using said chlorine atom and bromine atom, a preferred reactivity may be ensured. The halogen atom X² is more preferably a chlorine atom. By using said chlorine atom, a more preferred reactivity may be ensured.

Specific examples of the 1-halo-3,7-dimethyldecane compound (8) include 1-chloro-3,7-dimethyldecane, 1-bromo-3,7-dimethyldecane, and 1-iodo-3,7-dimethyldecane. The 1-halo-3,7-dimethyldecane compound (8) is preferably 1-chloro-3,7-dimethyldecane and 1-bromo-3,7-dimethyldecane. By using said 1-chloro-3,7-dimethyldecane and 1-bromo-3,7-dimethyldecane, a preferred reactivity may be ensured. The 1-halo-3,7-dimethyldecane compound (8) is more preferably 1-chloro-3,7-dimethyldecane. By using said 1-chloro-3,7-dimethyldecane, a more preferred reactivity may be ensured.

Next, a process for preparing the nucleophilic reagent, 3,7-dimethyldecyl compound (2), when M¹ is MgZ¹, that is, the 3,7-dimethyldecylmagnesium halide reagent (2: M¹ = MgZ¹) (i.e., a Grignard reagent), will be described in detail as an example.

The Grignard reagent, 3,7-dimethyldecylmagnesium halide reagent (2: M¹ = MgZ¹), may be prepared, for example, by reacting the aforesaid 1-halo-3,7-dimethyldecane compound (8) with magnesium in a solvent, as shown by the following chemical reaction formula:

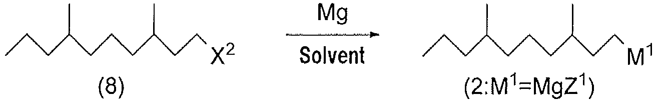

When preparing the 3,7-dimethyldecylmagnesium halide reagent (2), the amount of magnesium used, per mol of the 1-halo-3,7-dimethyldecane compound (8), is preferably 1.0 to 2.0 gram atoms. By using said preferred amount, a preferred completion of the reaction may be ensured.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; and hydrocarbon solvents such as hexane, heptane, benzene, toluene, xylene, and cumene. The solvent is preferably hydrocarbon solvents such as toluene and xylene; and ether solvents such as tetrahydrofuran, 2-methyltetrahydrofuran, and 4-methyltetrahydropyran. By using said hydrocarbon solvents such as toluene and xylene; and ether solvents such as tetrahydrofuran, 2-methyltetrahydrofuran, and 4-methyltetrahydropyran, a preferred reaction rate of forming the aforesaid Grignard reagent may be ensured. The solvent is more preferably tetrahydrofuran, 2-methyltetrahydrofuran, and 4-methyltetrahydropyran. By using said tetrahydrofuran, 2-methyltetrahydrofuran, and 4-methyltetrahydropyran, a more preferred reaction rate of forming the aforesaid Grignard reagent may be ensured.

The solvent may be used alone or in combination thereof, if necessary. The solvent may be a commercially available one.

The amount of the solvent used, per mol of the 1-halo-3,7-dimethyldecane compound (8), is preferably 30 to 5,000 g, more preferably 50 to 3,000 g, and even more preferably 100 to 1,000 g. By using said preferred amount, said more preferred amount, and said even more preferred amount, a preferred reactivity, a more preferred reactivity, and an even more preferred reactivity may be ensured.

The reaction temperature varies, depending on the solvent used, and is preferably 30 to 120°C, more preferably 50 to 100°C, and even more preferably 60 to 80°C. By using said preferred reaction temperature, said more preferred reaction temperature, and said even more preferred reaction temperature, a preferred reactivity, a more preferred reactivity, and an even more preferred reactivity may be ensured.

The reaction time varies, depending on the solvent used and/or the production scale, and is preferably 0.5 to 100 hours. By using said preferred reaction time, a preferred reactivity may be ensured.

(c) A nucleophilic addition reaction of a nucleophilic reagent, 3,7-dimethyldecyl compound (2), and propylene oxide (3) will be explained in detail below.

The amount of the propylene oxide (3) used in the nucleophilic addition reaction, per mol of the nucleophilic reagent, 3,7-dimethyldecyl compound (2), is preferably 0.8 to 2.0 mol, more preferably 0.9 to 1.7 mol, and even more preferably 1.0 to 1.5 mol. By using said preferred amount, said more preferred amount, and said even more preferred amount, preferred economy, more preferred economy, and even more preferred economy may be ensured.

A solvent may be incorporated in the nucleophilic addition reaction, if necessary. Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N*,*N-*dimethylformamide (DMF), *N*,*N*-dimethylacetamide (DMAC), *N*-methylpyrrolidone (NMP), dimethyl sulfoxide (DMSO), γ-butyrolactone (GBL), acetonitrile, *N*,*N'*-dimethylpropylene urea (DMPU), hexamethylphosphoric triamide (HMPA), dichloromethane, and chloroform. The solvent is preferably hydrocarbon solvents such as toluene and xylene; and ether solvents such as tetrahydrofuran, 2-methyltetrahydrofuran, and 4-methyltetrahydropyran. By using said hydrocarbon solvents such as toluene and xylene; and ether solvents such as tetrahydrofuran, 2-methyltetrahydrofuran, and 4-methyltetrahydropyran, a preferred reactivity may be ensured. The solvent is more preferably tetrahydrofuran, 2-methyltetrahydrofuran, and 4-methyltetrahydropyran. By using said tetrahydrofuran, 2-methyltetrahydrofuran, and 4-methyltetrahydropyran, a more preferred reactivity may be ensured.

The solvent may be used alone or in combination thereof, if necessary. The solvent may be a commercially available one.

When a solvent is incorporated in the nucleophilic addition reaction, the amount of the solvent used, per mol of the nucleophilic reagent, 3,7-dimethyldecyl compound (2), is preferably 30 to 5,000 g, more preferably 50 to 3,000 g, and even more preferably 100 to 1,000 g. By using said preferred amount, said more preferred amount, and said even more preferred amount, a preferred reactivity, a more preferred reactivity, and an even more preferred reactivity may be ensured.

The nucleophilic addition reaction may be carried out in the presence of a catalyst, if necessary.

Examples of the catalyst include copper compounds such as cuprous halides such as cuprous chloride, cuprous bromide, and cuprous iodide, and cupric halides such as cupric chloride, cupric bromide, and cupric iodide. The catalyst is preferably cuprous halides. By using said cuprous halides, a preferred reactivity may be ensured. The catalyst is more preferably cuprous chloride, By using said cuprous chloride, a more preferred reactivity may be ensured.

The catalyst may be used alone or in combination thereof, if necessary. The catalyst may be a commercially available one.

The amount of the catalyst used, per mol of the nucleophilic reagent, 3,7-dimethyldecyl compound (2), is preferably 0.0001 to 1.0 mol, more preferably 0.0005 to 0.3 mol, and even more preferably 0.001 to 0.1 mol. By using said preferred amount, said more preferred amount, and said even more preferred amount, a preferred reaction rate and/or post-treatment, a more preferred reaction rate and/or post-treatment, and an even more preferred reaction rate and/or post-treatment may be ensured.

The reaction temperature of the nucleophilic addition reaction varies, depending on the nucleophilic reagent, 3,7-dimethyldecyl compound (2), used, and is preferably -78 to 70°C, more preferably -20 to 50°C, and even more preferably 0 to 30°C. By using said preferred reaction temperature, said more preferred reaction temperature, and said even more preferred reaction temperature, a preferred reactivity, a more preferred reactivity, and an even more preferred reactivity may be ensured.

The reaction time of the nucleophilic addition reaction varies, depending on the solvent used and/or production scale, and is preferably 0.5 to 100 hours. By using said preferred reaction time, a preferred reactivity may be ensured.

Examples of the purification method of the nucleophilic addition reaction include purification by silica gel column chromatography and purification by distillation. The aforesaid purification method is preferably purification by distillation. By using said purification by distillation, a preferred industrial production may be ensured.

### (III) 1-Halo-3,7-dimethyldecane compound (8) and process for preparing 1-halo-3,7-dimethyldecane compound (8)

A 1-halo-3,7-dimethyldecane compound (8) may be prepared, for example, by converting a 1-halo-2-methylpentane compound (12) into a nucleophilic reagent, 2-methylpentyl compound (6), and then subjecting the nucleophilic reagent, 2-methylpentyl compound (6), to a coupling reaction with a sulfonate compound (7), as shown by the following chemical reaction formula:

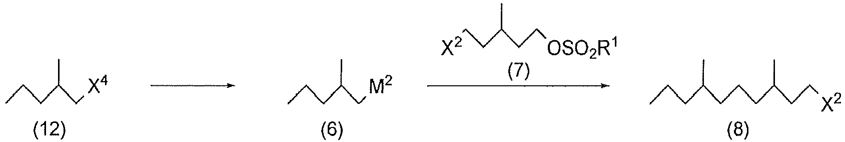

(a) First, the aforesaid nucleophilic reagent, 2-methylpentyl compound (6), will be explained in detail below. The nucleophilic reagent, 2-methylpentyl compound (6), is represented by the following general formula (6):

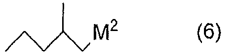

In the general formula (6) above, M² represents Li, MgZ², CuZ², or CuLiZ², and Z² represents a halogen atom or a 2-methylpentyl group. Specific examples of the halogen atom Z² include a chlorine atom, a bromine atom, and an iodine atom. The halogen atom Z² is preferably a chlorine atom and a bromine atom. By using said chlorine atom and bromine atom, a preferred reactivity may be ensured. The halogen atom Z² is more preferably a chlorine atom. By using said chlorine atom, a more preferred reactivity may be ensured.

Specific examples of the nucleophilic reagent, 2-methylpentyl compound (6), include the following compounds:
2-methylpentyllithium (when M² = Li);
2-methylpentylmagnesium halide compounds (when M² = MgZ²) such as 2-methylpentylmagnesium chloride, 2-methylpentylmagnesium bromide, and 2-methylpentylmagnesium iodide (i.e., Grignard reagents);
bis(2-methylpentyl)cuprate compounds (when M² = CuZ²) such as bis(2-methylpentyl)cuprate; and
Gilman reagents (when M² = CuLiZ²) such as lithium bis(2-methylpentyl)cuprate compounds such as lithium bis(2-methylpentyl)cuprate (i.e., Gilman reagents).
Grignard reagents such as 2-methylpentylmagnesium halide compounds are preferred. By using said Grignard reagents such as 2-methylpentylmagnesium halide compounds, a preferred reactivity may be ensured.

(b) A process for preparing the aforesaid nucleophilic reagent, 2-methylpentyl compound (6), will be explained in detail below.

A nucleophilic reagent, 2-methylpentyl compound (6), may be prepared from a 1-halo-2-methylpentane compound (12) below.

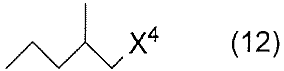

In the general formula (12) above, X⁴ represents a halogen atom. Specific examples of the halogen atom X⁴ include a chlorine atom, a bromine atom, and an iodine atom. The halogen atom X⁴ is preferably a chlorine atom and a bromine atom. By using said chlorine atom and bromine atom, a preferred reactivity may be ensured. The halogen atom X⁴ is more preferably a chlorine atom. By using said chlorine atom, a more preferred reactivity may be ensured.

Specific examples of the 1-halo-2-methylpentane compound (12) include 1-chloro-2-methylpentane, 1-bromo-2-methylpentane, and 1-iodo-2-methylpentane. 1-Chloro-2-methylpentane and 1-bromo-2-methylpentane are preferred. By using said 1-chloro-2-methylpentane and 1-bromo-2-methylpentane, a preferred reactivity may be ensured. 1-Chloro-2-methylpentane is more preferred. By using said 1-chloro-2-methylpentane, a more preferred reactivity may be ensured.

Next, a process for preparing the nucleophilic reagent, 2-methylpentyl compound (6), when M² is MgZ², that is, 2-methylpentylmagnesium halide reagent (6: M² = MgZ²) (i.e., a Grignard reagent), will be described in detail as an example.

The Grignard reagent, 2-methylpentylmagnesium halide reagent (6: M² = MgZ²), may be prepared, for example, by reacting the aforesaid 1-halo-2-methylpentane compound (12) with magnesium in a solvent, as shown by the following chemical reaction formula:

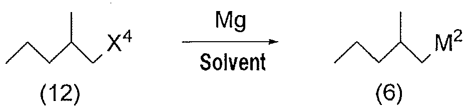

When preparing the 2-methylpentylmagnesium halide reagent (6), the amount of magnesium used, per mol of the 1-halo-2-methylpentane compound (12), is preferably 1.0 to 2.0 gram atoms. By using said preferred amount, a preferred completion of the reaction may be ensured.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; and hydrocarbon solvents such as hexane, heptane, benzene, toluene, xylene, and cumene. The solvent is preferably hydrocarbon solvents such as toluene and xylene; and ether solvents such as tetrahydrofuran, 2-methyltetrahydrofuran, and 4-methyltetrahydropyran. By using said hydrocarbon solvents such as toluene and xylene; and ether solvents such as tetrahydrofuran, 2-methyltetrahydrofuran, and 4-methyltetrahydropyran, a preferred reaction rate of forming the aforesaid Grignard reagent may be ensured. The solvent is more preferably tetrahydrofuran, 2-methyltetrahydrofuran, and 4-methyltetrahydropyran. By using said tetrahydrofuran, 2-methyltetrahydrofuran, and 4-methyltetrahydropyran, a more preferred reaction rate of forming the aforesaid Grignard reagent may be ensured.

The solvent may be used alone or in combination thereof, if necessary. The solvent may be a commercially available one.

The amount of the solvent used, per mol of the 1-halo-2-methylpentane compound (12), is preferably 30 to 5,000 g, more preferably 50 to 3,000 g, and even more preferably 100 to 1,000 g. By using said preferred amount, said more preferred amount, and said even more preferred amount, a preferred reactivity, a more preferred reactivity, and an even more preferred reactivity may be ensured.

The reaction temperature varies, depending on the solvent used, and is preferably 30 to 120°C, more preferably 50 to 100°C, and even more preferably 60 to 80°C. By using said preferred reaction temperature, said more preferred reaction temperature, and said even more preferred reaction temperature, a preferred reactivity, a more preferred reactivity, and an even more preferred reactivity may be ensured.

The reaction time varies, depending on the solvent used and/or production scale, and is preferably 0.5 to 100 hours. By using said preferred reaction time, a preferred reactivity may be ensured.

(c) A coupling reaction of a nucleophilic reagent, 2-methylpentyl compound (6), and a sulfonate compound (7) will be explained in detail below.

A sulfonate compound (7) is represented by the following general formula (7):

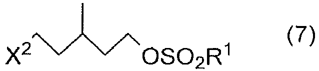

In the general formula (7) above, X² represents a halogen atom. Specific examples of the halogen atom X² include a chlorine atom, a bromine atom, and an iodine atom. The halogen atom X² is preferably a chlorine atom and a bromine atom. By using said chlorine atom and bromine atom, a preferred reactivity may be ensured. The halogen atom X² is more preferably a chlorine atom. By using said chlorine atom, a more preferred reactivity may be ensured.

R¹ represents a hydrocarbon group having 1 to 7 carbon atoms. Examples of the hydrocarbon group having 1 to 7 carbon atoms include a linear or branched alkyl group having 1 to 5 carbon atoms, and preferably 1 to 4 carbon atoms; and an aryl group having 6 to 7 carbon atoms.

Examples of the linear or branched alkyl group having 1 to 5 carbon atoms include a methyl group, an ethyl group, an n-propyl group, an isopropyl group, an n-butyl group, a 2-butyl group, and an n-pentyl group. Examples of the aryl group having 6 to 7 carbon atoms include a phenyl group and a tolyl group. The hydrocarbon group having 1 to 7 carbon atoms is preferably a phenyl group and a tolyl group. By using said phenyl group and tolyl group, a preferred reactivity may be ensured. The hydrocarbon group having 1 to 7 carbon atoms is more preferably a phenyl group. By using said phenyl group, a more preferred reactivity may be ensured.

Specific examples of the sulfonate compound (7) include the following compounds:
5-halo-3-methyl methanesulfonate compounds such as 5-chloro-3-methyl methanesulfonate, 5-bromo-3-methyl methanesulfonate, and 5-iodo-3-methyl methanesulfonate;
5-halo-3-methyl benzenesulfonate compounds such as 5-chloro-3-methyl benzenesulfonate, 5-bromo-3-methyl benzenesulfonate, and 5-iodo-3-methyl benzenesulfonate; and
5-halo-3-methyl *p*-toluenesulfonate compounds such as 5-chloro-3-methyl *p-*toluenesulfonate, 5-bromo-3-methyl *p*-toluenesulfonate, and 5-iodo-3-methyl *p-*toluenesulfonate.

The sulfonate compound (7) is preferably 5-chloro-3-methyl methanesulfonate, 5-bromo-3-methyl methanesulfonate, 5-iodo-3-methyl methanesulfonate, 5-chloro-3-methyl benzenesulfonate, 5-bromo-3-methyl benzenesulfonate, 5-iodo-3-methyl benzenesulfonate, 5-chloro-3-methyl *p*-toluenesulfonate, 5-bromo-3-methyl *p-*toluenesulfonate, and 5-iodo-3-methyl *p*-toluenesulfonate. By using said 5-chloro-3-methyl methanesulfonate, 5-bromo-3-methyl methanesulfonate, 5-iodo-3-methyl methanesulfonate, 5-chloro-3-methyl benzenesulfonate, 5-bromo-3-methyl benzenesulfonate, 5-iodo-3-methyl benzenesulfonate, 5-chloro-3-methyl *p-*toluenesulfonate, 5-bromo-3-methyl *p*-toluenesulfonate, and 5-iodo-3-methyl *p-*toluenesulfonate, a preferred reactivity may be ensured. The sulfonate compound (7) is more preferably 5-chloro-3-methyl methanesulfonate, 5-bromo-3-methyl methanesulfonate, 5-chloro-3-methyl benzenesulfonate, 5-bromo-3-methyl benzenesulfonate, 5-chloro-3-methyl *p*-toluenesulfonate, and 5-bromo-3-methyl *p-*toluenesulfonate. By using said 5-chloro-3-methyl methanesulfonate, 5-bromo-3-methyl methanesulfonate, 5-chloro-3-methyl benzenesulfonate, 5-bromo-3-methyl benzenesulfonate, 5-chloro-3-methyl *p*-toluenesulfonate, and 5-bromo-3-methyl *p-*toluenesulfonate, a more preferred reactivity may be ensured. The sulfonate compound (7) is even more preferably 5-chloro-3-methyl methanesulfonate, 5-chloro-3-methyl benzenesulfonate, and 5-chloro-3-methyl *p*-toluenesulfonate. By using said 5-chloro-3-methyl methanesulfonate, 5-chloro-3-methyl benzenesulfonate, and 5-chloro-3-methyl*p-*toluenesulfonate, an even more preferred reactivity may be ensured. The sulfonate compound (7) is particularly preferably 5-chloro-3-methyl benzenesulfonate. By using said 5-chloro-3-methyl benzenesulfonate, a particularly preferred reactivity may be ensured.

The amount of the sulfonate compound (7) used in the coupling reaction, per mol of the nucleophilic reagent, 2-methylpentyl compound (6), is preferably 0.7 to 2.0 mol, more preferably 0.8 to 1.7 mol, and even more preferably 0.9 to 1.4 mol. By using said preferred amount, said more preferred amount, and said even more preferred amount, preferred economy, more preferred economy, and even more preferred economy may be ensured.

A solvent may be incorporated in the coupling reaction, if necessary. Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N*,*N*-dimethylformamide (DMF), *N*,*N*-dimethylacetamide (DMAC), *N*-methylpyrrolidone (NMP), dimethyl sulfoxide (DMSO), γ-butyrolactone (GBL), acetonitrile, *N*,*N'*-dimethylpropylene urea (DMPU), hexamethylphosphoric triamide (HMPA), dichloromethane, and chloroform. The solvent is preferably hydrocarbon solvents such as toluene and xylene; and ether solvents such as tetrahydrofuran, 2-methyltetrahydrofuran, and 4-methyltetrahydropyran. By using said hydrocarbon solvents such as toluene and xylene; and ether solvents such as tetrahydrofuran, 2-methyltetrahydrofuran, and 4-methyltetrahydropyran, a preferred reactivity may be ensured. The solvent is more preferably tetrahydrofuran, 2-methyltetrahydrofuran, and 4-methyltetrahydropyran. By using said tetrahydrofuran, 2-methyltetrahydrofuran, and 4-methyltetrahydropyran, a more preferred reactivity may be ensured.

The solvent may be used alone or in combination thereof, if necessary. The solvent may be a commercially available one.

The amount of the solvent used, per mol of the nucleophilic reagent, 2-methylpentyl compound (6), is preferably 30 to 5,000 g, more preferably 50 to 3,000 g, and even more preferably 100 to 1,000 g. By using said preferred amount, said more preferred amount, and said even more preferred amount, a preferred reactivity, a more preferred reactivity, and an even more preferred reactivity may be ensured.

The coupling reaction of the nucleophilic reagent, 2-methylpentyl compound (6), with the sulfonate compound (7) may be carried out in the presence of a catalyst, if necessary.

Examples of the catalyst include copper compounds such as cuprous halides such as cuprous chloride, cuprous bromide, and cuprous iodide, and cupric halides such as cupric chloride, cupric bromide, and cupric iodide; iron compounds such as iron(II) chloride, iron(III) chloride, iron(II) bromide, iron(III) bromide, iron(II) iodide, iron(III) iodide, and iron(III) acetylacetonate; silver compounds such as silver chloride, silver nitrate, and silver acetate; titanium compounds such as titanium tetrachloride, titanium tetrabromide, titanium(IV) methoxide, titanium(IV) ethoxide, titanium(IV) isopropoxide, and titanium(IV) oxide; palladium(II) compounds such as dichlorobis(triphenylphosphine)palladium and dichloro[1,1'-bis(diphenylphosphino)ferrocene]palladium; and nickel compounds such as nickel chloride, dichloro[1,2-bis(diphenylphosphino)ethane]nickel(II), and dichlorobis(triphenylphosphine)nickel(II). The catalyst is preferably copper compounds. By using said copper compounds, a preferred reactivity and/or economy may be ensured. The catalyst is more preferably cupric halides such as cupric chloride, cupric bromide, and cupric iodide. By using said cupric halides such as cupric chloride, cupric bromide, and cupric iodide, a more preferred reactivity and/or economy may be ensured.

The catalyst may be used alone or in combination thereof, if necessary. The catalyst may be a commercially available one.

The amount of the catalyst used, per mol of the 1-halo-2-methylpentane compound (12), is preferably 0.0001 to 1.0 mol, more preferably 0.0005 to 0.5 mol, and even more preferably 0.001 to 0.3 mol. By using said preferred amount, said more preferred amount, and said even more preferred amount, a preferred reaction rate and/or post-treatment, a more preferred reaction rate and/or post-treatment, and an even more preferred reaction rate and/or post-treatment may be ensured.

When the coupling reaction is carried out in the presence of a catalyst, a co-catalyst may be used, if necessary. Examples of the co-catalyst include trialkyl phosphite compounds having 3 to 9 carbon atoms such as triethyl phosphite; and arylphosphine compounds having 18 to 44 carbon atoms such as triphenylphosphine, tritolylphosphine, and 2,2'-bis(diphenylphosphino)-1,1'-binaphthyl (BINAP). The co-catalyst is preferably trialkyl phosphite. By using said trialkyl phosphite, a preferred reactivity may be ensured. The co-catalyst is particularly preferably triethyl phosphite. By using said triethyl phosphite, a particularly preferred reactivity may be ensured.

The co-catalyst may be used alone or in combination thereof, if necessary. The co-catalyst may be a commercially available one.

The amount of the co-catalyst used, per mol of the 1-halo-2-methylpentane compound (12), is preferably 0.0001 to 1.0 mol, more preferably 0.0005 to 0.5 mol, and even more preferably 0.001 to 0.3 mol.

When the coupling reaction is carried out in the presence of a catalyst, a lithium salt may be added, if necessary. The lithium salt may be incorporated in the coupling reaction with the aforesaid co-catalyst, or the lithium salt may be incorporated alone without the aforesaid co-catalyst. Examples of the lithium salt include lithium halides such as lithium chloride, lithium bromide, and lithium iodide; lithium nitrate; and lithium carbonate. The lithium salt is preferably lithium halides such as lithium chloride; and lithium nitrate. By using said lithium halides such as lithium chloride; and lithium nitrate, a preferred reactivity may be ensured.

The lithium salt may be used alone or in combination thereof, if necessary. The lithium salt may be a commercially available one.

The amount of the lithium salt used in the coupling reaction, per mol of the 1-halo-2-methylpentane compound (12), is preferably 0.0001 to 1.0 mol, more preferably 0.0005 to 0.5 mol, and even more preferably 0.001 to 0.3 mol. By using said preferred amount, said more preferred amount, and said even more preferred amount, a preferred reactivity, a more preferred reactivity, and an even more preferred reactivity may be ensured.

The reaction temperature of the coupling reaction varies, depending on the nucleophilic reagent, 2-methylpentyl compound (6), used, and is preferably -78 to 100°C, more preferably -25 to 70°C, even more preferably -10 to 50°C, and particularly preferably 10 to 35°C. By using said preferred reaction temperature, said more preferred reaction temperature, said even more preferred reaction temperature, and said particularly preferred reaction temperature, a preferred reactivity, a more preferred reactivity, an even more preferred reactivity, and a particularly preferred reactivity may be ensured.

The reaction time of the coupling reaction varies, depending on the solvent used and/or production scale, and is preferably 0.5 to 100 hours. By using said preferred reaction time, a preferred reactivity may be ensured.

Examples of the purification method of the coupling reaction include purification by silica gel column chromatography and purification by distillation. By using said purification by distillation, a preferred industrial production may be ensured.

(IV) Synthesis of an alkyl 2,6,10-trimethyltridecanoate compound (11) by converting a 2-halo-6,10-dimethyltridecane compound (1) into a nucleophilic reagent, 1,5,9-trimethyldodecyl compound (9), and then subjecting the nucleophilic reagent, 1,5,9-trimethyldodecyl compound (9), to a nucleophilic substitution reaction with alkyl haloformate (10) will be explained in detail below.

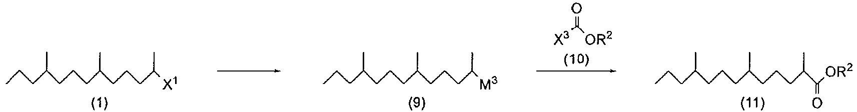

(a) First, the aforesaid nucleophilic reagent, 1,5,9-trimethyldodecyl compound (9), will be explained in detail below. The nucleophilic reagent, 1,5,9-trimethyldodecyl compound (9), is represented by the following general formula (9):

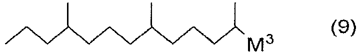

In the general formula (9) above, M³ represents Li, MgZ³, CuZ³, or CuLiZ³, and Z³ represents a halogen atom or a 1,5,9-trimethyldodecyl group. Specific examples of the halogen atom Z³ include a chlorine atom, a bromine atom, and an iodine atom. The halogen atom Z³ is preferably a chlorine atom and a bromine atom. By using said chlorine atom and bromine atom, a preferred reactivity may be ensured. The halogen atom Z³ is more preferably a chlorine atom. By using said chlorine atom, a more preferred reactivity may be ensured.

Specific examples of the nucleophilic reagent, 1,5,9-trimethyldodecyl compound (9), include the following compounds:
1,5,9-trimethyldodecyllithium compounds (when M³ = Li) such as 1,5,9-trimethyldodecyllithium;
1,5,9-trimethyldodecylmagnesium halide compounds (when M³ = MgZ³) such as 1,5,9-trimethyldodecylmagnesium chloride, 1,5,9-trimethyldodecylmagnesium bromide, and 1,5,9-trimethyldodecylmagnesium iodide (i.e., Grignard reagents);
bis(1,5,9-trimethyldodecyl)cuprate compounds (when M³ = CuZ³) such as bis(1,5,9-trimethyldodecyl)cuprate; and
Gilman reagents (when M³ = CuLiZ³) such as lithium bis(1,5,9-trimethyldodecyl)cuprate compounds such as lithium bis(1,5,9-trimethyldodecyl)cuprate (i.e., Gilman reagents).
Grignard reagents such as 1,5,9-trimethyldodecylmagnesium halide compounds are preferred. By using said Grignard reagents such as 1,5,9-trimethyldodecylmagnesium halide compounds, a preferred reactivity may be ensured.

(b) A process for preparing the aforesaid nucleophilic reagent, 1,5,9-trimethyldodecyl compound (9), will be explained in detail below.

The nucleophilic reagent, 1,5,9-trimethyldodecyl compound (9), may be prepared from the aforesaid 2-halo-6,10-dimethyltridecane compound (1).

Next, a process for preparing the nucleophilic reagent, 1,5,9-trimethyldodecyl compound (9), when M³ is MgZ³, that is, 1,5,9-trimethyldodecylmagnesium halide reagent (9: M³ = MgZ³) (i.e., a Grignard reagent), will be described in detail as an example.

The Grignard reagent, 1,5,9-trimethyldodecylmagnesium halide reagent (9: M³ = MgZ³), may be prepared, for example, by reacting the aforesaid 2-halo-6,10-dimethyltridecane compound (1) with magnesium in a solvent, as shown by the following chemical reaction formula:

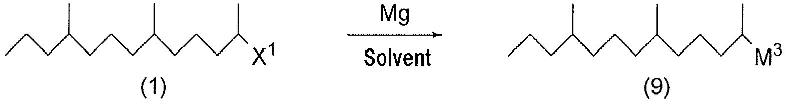

When preparing the nucleophilic reagent, 1,5,9-trimethyldodecyl compound (9), the amount of magnesium used, per mol of the 2-halo-6,10-dimethyltridecane compound (1), is preferably 1.0 to 2.0 gram atoms. By using said preferred amount, a preferred completion of the reaction may be ensured.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; and hydrocarbon solvents such as hexane, heptane, benzene, toluene, xylene, and cumene. The solvent is preferably hydrocarbon solvents such as toluene and xylene; and ether solvents such as tetrahydrofuran, 2-methyltetrahydrofuran, and 4-methyltetrahydropyran. By using said hydrocarbon solvents such as toluene and xylene; and ether solvents such as tetrahydrofuran, 2-methyltetrahydrofuran, and 4-methyltetrahydropyran, a preferred reaction rate of forming the aforesaid Grignard reagent may be ensured. The solvent is more preferably tetrahydrofuran, 2-methyltetrahydrofuran, and 4-methyltetrahydropyran. By using said tetrahydrofuran, 2-methyltetrahydrofuran, and 4-methyltetrahydropyran, a more preferred reaction rate of forming the aforesaid Grignard reagent may be ensured.

The solvent may be used alone or in combination thereof, if necessary. The solvent may be a commercially available one.

The amount of the solvent used, per mol of the 2-halo-6,10-dimethyltridecane compound (1), is preferably 30 to 5,000 g, more preferably 50 to 3,000 g, and even more preferably 100 to 1,000 g. By using said preferred amount, said more preferred amount, and said even more preferred amount, a preferred reactivity, a more preferred reactivity, and an even more preferred reactivity may be ensured.

The reaction temperature varies, depending on the solvent used, and is preferably 30 to 120°C, more preferably 50 to 100°C, and even more preferably 60 to 80°C. By using said preferred reaction temperature, said more preferred reaction temperature, and said even more preferred reaction temperature, a preferred reactivity, a more preferred reactivity, and an even more preferred reactivity may be ensured.

The reaction time varies, depending on the solvent used and/or production scale, and is preferably 0.5 to 100 hours. By using said preferred reaction time, a preferred reactivity may be ensured.

(c) A process for preparing an alkyl 2,6,10-trimethyltridecanoate compound (11) by subjecting a nucleophilic reagent, 1,5,9-trimethyldodecyl compound (9), to a nucleophilic substitution reaction with an alkyl haloformate (10) will be explained in detail below.

The aforesaid alkyl haloformate (10) is represented by the following general formula (10):

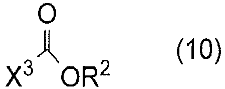

In the general formula (10) above, X³ represents a halogen atom. Specific examples of the halogen atom X³ include a chlorine atom, a bromine atom, and an iodine atom. The halogen atom X³ is preferably a chlorine atom and a bromine atom. By using said chlorine atom and bromine atom, a preferred reactivity may be ensured. The halogen atom X³ is more preferably a chlorine atom. By using said chlorine atom, a more preferred reactivity may be ensured.

R² represents an alkyl group having 1 to 12 carbon atoms, preferably 1 to 10 carbon atoms, more preferably 1 to 6 carbon atoms, even more preferably 1 to 4 carbon atoms, still more preferably 1 to 2 carbon atoms, and particularly preferably 1 carbon atom.

Specific examples of the alkyl group having 1 to 12 carbon atoms include a methyl group, an ethyl group, an n-propyl group, a butyl group, a pentyl group, a hexyl group, a heptyl group, an octyl group, a nonyl group, a decyl group, an undecyl group, and a dodecyl group.

The alkyl group is preferably a methyl group, an ethyl group, a hexyl group, and a decyl group, more preferably a methyl group and an ethyl group, and even more preferably a methyl group.

Specific examples of the alkyl haloformate (10) include the following:
alkyl chloroformate compounds such as methyl chloroformate, ethyl chloroformate, propyl chloroformate, isopropyl chloroformate, butyl chloroformate, pentyl chloroformate, hexyl chloroformate, heptyl chloroformate, octyl chloroformate, nonyl chloroformate, decyl chloroformate, undecyl chloroformate, and dodecyl chloroformate;
alkyl bromoformate compounds such as methyl bromoformate, ethyl bromoformate, propyl bromoformate, isopropyl bromoformate, butyl bromoformate, pentyl bromoformate, hexyl bromoformate, heptyl bromoformate, octyl bromoformate, nonyl bromoformate, decyl bromoformate, undecyl bromoformate, and dodecyl bromoformate; and
alkyl iodoformate compounds such as methyl iodoformate, ethyl iodoformate, propyl iodoformate, isopropyl iodoformate, butyl iodoformate, pentyl iodoformate, hexyl iodoformate, heptyl iodoformate, octyl iodoformate, nonyl iodoformate, decyl iodoformate, undecyl iodoformate, and dodecyl iodoformate.

The aforesaid compound is preferably alkyl chloroformate compounds such as methyl chloroformate, ethyl chloroformate, propyl chloroformate, isopropyl chloroformate, butyl chloroformate, pentyl chloroformate, hexyl chloroformate, heptyl chloroformate, octyl chloroformate, nonyl chloroformate, decyl chloroformate, undecyl chloroformate, and dodecyl chloroformate; and alkyl bromoformate compounds such as methyl bromoformate, ethyl bromoformate, propyl bromoformate, isopropyl bromoformate, butyl bromoformate, pentyl bromoformate, hexyl bromoformate, heptyl bromoformate, octyl bromoformate, nonyl bromoformate, decyl bromoformate, undecyl bromoformate, and dodecyl bromoformate; more preferably alkyl chloroformate compounds such as methyl chloroformate, ethyl chloroformate, propyl chloroformate, isopropyl chloroformate, butyl chloroformate, pentyl chloroformate, hexyl chloroformate, heptyl chloroformate, octyl chloroformate, nonyl chloroformate, decyl chloroformate, undecyl chloroformate, and dodecyl chloroformate; even more preferably methyl chloroformate, ethyl chloroformate, propyl chloroformate, isopropyl chloroformate, butyl chloroformate, pentyl chloroformate, and hexyl chloroformate; still more preferably methyl chloroformate and ethyl chloroformate; and particularly preferably methyl chloroformate.

The aforesaid alkyl 2,6,10-trimethyltridecanoate compound (11) is represented by the following general formula (11):

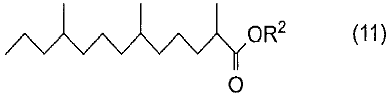

R² represents an alkyl group having 1 to 12 carbon atoms, preferably 1 to 10 carbon atoms, more preferably 1 to 6 carbon atoms, even more preferably 1 to 4 carbon atoms, still more preferably 1 to 2 carbon atoms, and particularly preferably 1 carbon atom.

Specific examples of the alkyl group having 1 to 12 carbon atoms include a methyl group, an ethyl group, an n-propyl group, a butyl group, a pentyl group, a hexyl group, a heptyl group, an octyl group, a nonyl group, a decyl group, an undecyl group, and a dodecyl group.

The alkyl group is preferably a methyl group, an ethyl group, a hexyl group, and a decyl group, more preferably a methyl group and an ethyl group, and even more preferably a methyl group.

Specific examples of the alkyl 2,6,10-trimethyltridecanoate compound (11) include alkyl 2,6,10-trimethyltridecanoate compounds such as methyl 2,6,10-trimethyltridecanoate, ethyl 2,6,10-trimethyltridecanoate, propyl 2,6,10-trimethyltridecanoate, butyl 2,6,10-trimethyltridecanoate, pentyl 2,6,10-trimethyltridecanoate, hexyl 2,6,10-trimethyltridecanoate, heptyl 2,6,10-trimethyltridecanoate, octyl 2,6,10-trimethyltridecanoate, nonyl 2,6,10-trimethyltridecanoate, decyl 2,6,10-trimethyltridecanoate, undecyl 2,6,10-trimethyltridecanoate, and dodecyl 2,6,10-trimethyltridecanoate.

The aforesaid compounds are preferably methyl 2,6,10-trimethyltridecanoate, ethyl 2,6,10-trimethyltridecanoate, hexyl 2,6,10-trimethyltridecanoate, and decyl 2,6,10-trimethyltridecanoate; more preferably methyl 2,6,10-trimethyltridecanoate and ethyl 2,6,10-trimethyltridecanoate; and even more preferably methyl 2,6,10-trimethyltridecanoate.

The amount of the alkyl haloformate (10) used in the aforesaid nucleophilic substitution reaction, per mol of the nucleophilic reagent, 1,5,9-trimethyldodecyl compound (9), is preferably 0.7 to 2.0 mol, more preferably 0.8 to 1.7 mol, and even more preferably 0.9 to 1.4 mol. By using said preferred amount, said more preferred amount, and said even more preferred amount, preferred economy, more preferred economy, and even more preferred economy may be ensured.

A solvent may be incorporated in the nucleophilic substitution reaction, if necessary. Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N*,*N*-dimethylformamide (DMF), *N*,*N-*dimethylacetamide (DMAC), *N*-methylpyrrolidone (NMP), dimethyl sulfoxide (DMSO), γ-butyrolactone (GBL), acetonitrile, *N*,*N'*-dimethylpropylene urea (DMPU), hexamethylphosphoric triamide (HMPA), dichloromethane, and chloroform. The solvent is preferably hydrocarbon solvents such as toluene and xylene; and ether solvents such as tetrahydrofuran, 2-methyltetrahydrofuran, and 4-methyltetrahydropyran. By using said hydrocarbon solvents such as toluene and xylene; and ether solvents such as tetrahydrofuran, 2-methyltetrahydrofuran, and 4-methyltetrahydropyran, a preferred reactivity may be ensured. The solvent is more preferably tetrahydrofuran, 2-methyltetrahydrofuran, and 4-methyltetrahydropyran. By using said tetrahydrofuran, 2-methyltetrahydrofuran, and 4-methyltetrahydropyran, a more preferred reactivity may be ensured.

The solvent may be used alone or in combination thereof, if necessary. The solvent may be a commercially available one.

The amount of the solvent used, per mol of the nucleophilic reagent, 1,5,9-trimethyldodecyl compound (9), is preferably 30 to 5,000 g, more preferably 50 to 3,000 g, and even more preferably 100 to 1,000 g. By using said preferred amount, said more preferred amount, and said even more preferred amount, a preferred reactivity, a more preferred reactivity, and an even more preferred reactivity may be ensured.

A base may be incorporated in the nucleophilic substitution reaction, if necessary. Examples of the base include pyridine and 4-dimethylaminopyridine (DMAP). The base is preferably 4-dimethylaminopyridine. By using said 4-dimethylaminopyridine, a preferred reactivity may be ensured.

The base may be used alone or in combination thereof, if necessary. The base may be a commercially available one.

The amount of the base used in the nucleophilic substitution reaction, per mol of the 2-halo-6,10-dimethyltridecane compound (1), is preferably 0.0001 to 1.0 mol, and more preferably 0.001 to 0.3 mol. By using said preferred amount and said more preferred amount, a preferred reactivity and a more preferred reactivity may be ensured.

The reaction temperature of the nucleophilic substitution reaction varies, depending on the nucleophilic reagent, 1,5,9-trimethyldodecyl compound (9), used, and is preferably -78 to 100°C, more preferably -25 to 60°C, even more preferably -15 to 30°C, and particularly preferably -10 to 15°C. By using said preferred reaction temperature, said more preferred reaction temperature, said even more preferred reaction temperature, and said particularly preferred reaction temperature, a preferred reactivity, a more preferred reactivity, an even more preferred reactivity, and a particularly preferred reactivity may be ensured.

The reaction time of the nucleophilic substitution reaction varies, depending on the solvent used and/or production scale, and is preferably 0.5 to 100 hours. By using said preferred reaction time, a preferred reactivity may be ensured.

Examples of the purification method of the nucleophilic substitution reaction include purification by silica gel column chromatography and purification by distillation. The aforesaid purification method is preferably purification by distillation. By using said purification by distillation, a preferred industrial production may be ensured. Alkenes in the reaction mixture that were generated by elimination of hydrogen halide in a halogenation step before purification may be completely separated by the distillation of this step.

Thus, according to an embodiment of the present invention, the synthetic intermediate, 2-halo-6,10-dimethyltridecane compound (1), may be easily and efficiently prepared. According to an embodiment of the present invention, alkyl 2,6,10-trimethyltridecanoate compounds including the pheromone of the Neotropical brown stink bug (scientific name: *Euschistus heros*), methyl 2,6,10-trimethyltridecanoate, may be comprehensively prepared from a 2-halo-6,10-dimethyltridecane compound (1) with high ease of preparation and in fewer steps. Specifically, because the target compound may be selectively synthesized, alkyl 2,6,10-trimethyltridecanoate (11) may be efficiently prepared without the need for separation by column chromatography or the like, and alkyl 2,6,10-trimethyltridecanoate compounds may be made individually in the final step.

### EXAMPLES

The present invention will be described with reference to the following Examples. It should be noted that the present invention is not limited to or by the Examples.

The term "purity" as used herein means an area percentage in gas chromatography (GC), unless otherwise specified. The term "product ratio" means a ratio of area percentages in GC. The term "yield" is calculated from the area percentages determined by GC.

In the Examples, monitoring of the reactions and calculation of the yields were carried out in the following GC conditions.

GC conditions: GC: Capillary gas chromatograph GC-2014 (Shimadzu Corporation); column: DB-5, 0.25 µm x 0.25 mmϕ x 30 m; carrier gas: He (1.55 mL/min), detector: FID; column temperature: 100°C, elevated in a rate of 5°C/min, and up to 230°C.

The yield was calculated according to the following equation in consideration of purities (% GC) of a starting material and a product. 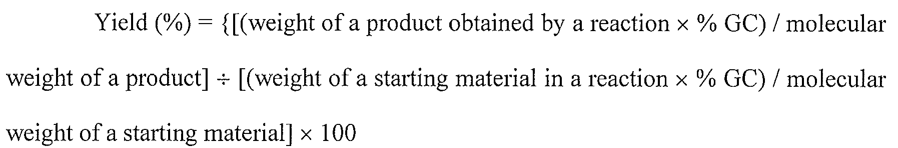 Yield (%) = {[(weight of a product obtained by a reaction × % GC) / molecular weight of a product] ÷ [(weight of a starting material in a reaction × % GC) / molecular weight of a starting material] × 100

THF represents tetrahydrofuran, Ph represents a phenyl group, and GBL represents γ-butyrolactone.

### Example 1: Preparation of 1-halo-3,7-dimethyldecane (8: X² = Cl)

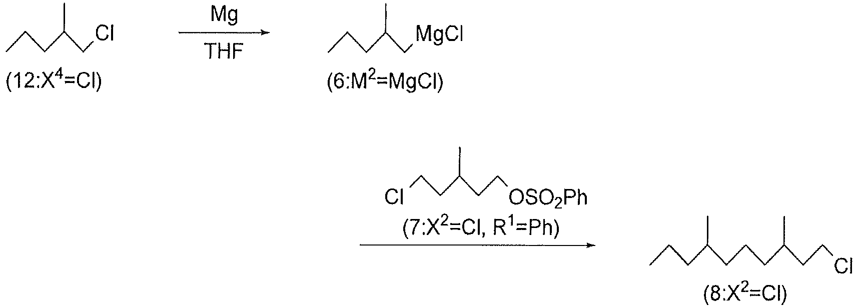

Magnesium (275.55 g, 11.34 gram atoms) and tetrahydrofuran (3,240.00 g) were placed in a reactor at a room temperature and stirred for 36 minutes at 60 to 65°C. A 1-chloro-2-methylpentane compound (12: X⁴ = Cl) (1,302.69 g, 10.80 mol, purity 100%) was then added dropwise to the reactor at 60 to 75°C. After the completion of the dropwise addition, the reaction mixture was stirred for 3 hours at 75 to 80°C to form 2-methylpentylmagnesium chloride (6: M² = MgCl).

Cupric chloride (7.26 g, 0.054 mol), lithium chloride (4.59 g, 0.11 mol), tetrahydrofuran (3,600.00 g), and 5-chloro-3-methylpentyl benzenesulfonate (7: X² = Cl, R¹ = Ph) (3,377.04 g, 10.26 mol, purity 84.09%) were then placed in another reactor, and then the aforesaid 2-methylpentylmagnesium chloride (6: M² = MgCl) was added dropwise to the other reactor at 15 to 25°C. After the completion of the dropwise addition, the reaction mixture was stirred for 4.5 hours at 20 to 30°C. Methanol (1,687.50 g) and then water (780.00 g) were added to the reaction mixture, followed by phase separation. The organic layer thus obtained was then concentrated at a reduced pressure, and the resulting concentrate was distilled at a reduced pressure to form 1-halo-3,7-dimethyldecane (8: X² = Cl) (1,657.24 g, 8.04 mol, purity 99.36%, b.p. = 90.1 to 96.1°C/0.044 kPa (0.33 mmHg)) with a yield of 78.37%. Although the concentrated solution of 1-halo-3,7-dimethyldecane (8: X² = Cl) thus obtained contained 4,8,12-trimethylpentadecane formed by a reaction of 1-halo-3,7-dimethyldecane (8: X² = Cl) with the 1-chloro-2-methylpentane compound (12: X⁴ = Cl), 4,8,12-trimethylpentadecane was separable by the aforesaid distillation, and the fraction of 4,8,12-trimethylpentadecane remaining after distillation was 0.5% or less.

The following is the spectrum data of the 1-halo-3,7-dimethyldecane (8: X² = Cl) thus obtained.

Nuclear magnetic resonance spectrum: ¹H-NMR (500 MHz, CDCl₃): δ = 0.83-0.86 (3H, m), 0.86-0.92 (6H, m), 1.12-1.43 (11H, m), 1.53-1.84 (3H, m), 3.50-3.62 (2H, m); ¹³C-NMR (125 MHz, CDCl₃): δ = 14.39, 19.05, 19.11, 19.60, 19.67, 20.12, 24.22, 30.37, 30.41, 32.44, 32.47, 36.89, 36.94, 37.20, 37.25, 39.34, 39.44, 39.76, 39.84, 43.37.

Mass spectrum: EI-mass spectrum (70 eV): m/z 170 (M⁺-34), 155, 141, 127, 113, 98, 85, 71, 57, 43, 29.

Infrared absorption spectrum: (D-ATR): v = 2957, 2928, 2871, 1463, 1379, 729, 659.

### Example 2: Preparation of 6,10-dimethyl-2-tridecanol (4)

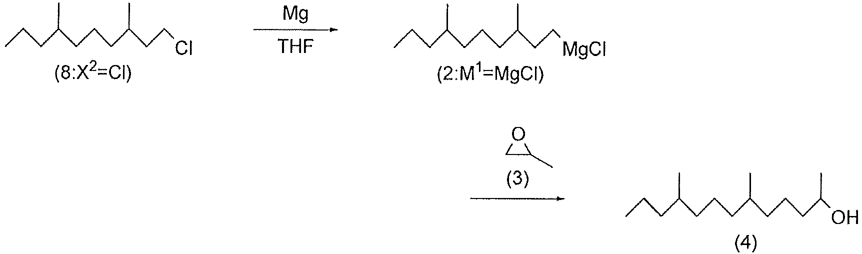

Magnesium (89.30 g, 3.68 gram atoms) and tetrahydrofuran (1,050.00 g) were placed in a reactor at a room temperature and then stirred for 18 minutes at 60 to 65°C. 1-Halo-3,7-dimethyldecane (8: X² = Cl) (721.35 g, 3.50 mol, purity 99.36%) prepared as in Example 1 was then added dropwise to the reactor at 60 to 75°C. After the completion of the dropwise addition, the reaction mixture was stirred for 3.5 hours at 70 to 75°C to form 3,7-dimethyldecylmagnesium chloride (2: M¹ = MgCl).

The internal temperature of the aforesaid reactor was then cooled to 0 to 10°C, after which cuprous chloride (0.73 g, 0.0070 mol, purity 95%) was added to the reactor, and then propylene oxide (233.77 g, 4.03 mol) was added dropwise at 5 to 30°C. After the completion of the dropwise addition, the reaction mixture was stirred for 2 hours at 15 to 25°C. An aqueous solution of acetic acid (prepared from acetic acid (477.67 g) and water (1,433.09 g)) and hexane (339.67 g) were then added to the reaction mixture, followed by phase separation. The organic layer thus obtained was then concentrated at a reduced pressure, and the resulting concentrate was distilled at a reduced pressure to form 6,10-dimethyl-2-tridecanol (4) (743.97 g, 3.19 mol, purity 97.85%, b.p. = 123.0 to 138.9°C/0.093 kPa (0.70 mmHg)) with a yield of 91.05%.

The following is the spectrum data of the 6,10-dimethyl-2-tridecanol (4) thus obtained.

Nuclear magnetic resonance spectrum: ¹H-NMR (500 MHz, CDCl₃): δ = 0.86 (9H, quin-like), 1.49 (1H, br.s), 1.00-1.48 (21H, m), 3.79 (1H, tq, J = 6.1 Hz, 6.1 Hz); ¹³C-NMR(125 MHz, CDCl₃): δ = 14.40, 19.62, 19.68, 20.13, 23.23, 23.46, 23.49, 24.43, 32.46, 32.48, 32.72, 32.74, 32.76, 36.99, 37.02, 37.08, 37.11, 37.31, 37.35, 37.40, 39.37, 39.47, 39.68, 39.71, 68.18, 68.21.

Mass spectrum: EI-mass spectrum (70 eV): m/z 227 (M⁺-1), 210, 182, 168, 154, 125, 111, 97, 84, 69, 57, 45, 29.

Infrared absorption spectrum: (D-ATR): v = 3334, 2958, 2927, 1463, 1377, 1120, 739.

### Example 3: Preparation of 2-chloro-6,10-dimethyltridecane (1: X¹ = Cl)

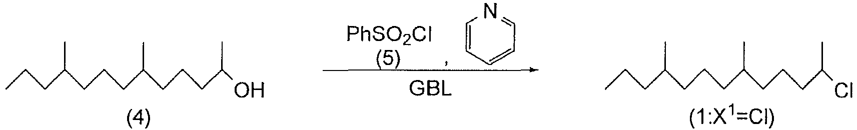

6,10-Dimethyl-2-tridecanol (4) (466.88 g, 2.00 mol, purity 97.85%) prepared as in Example 2, pyridine (237.30 g, 2.40 mol), and GBL (800.00 g) were placed in a reactor and then stirred for 42 minutes at 0 to 10°C.

Benzenesulfonyl chloride (PhSO₂Cl) (423.86 g, 3.00 mol) was then added dropwise at 0 to 10°C. After the completion of the dropwise addition, the reaction mixture was heated to 60 to 65°C and then stirred for 20 hours. After the completion of the stirring, water (600.00 g) and hexane (500.00 g) were added to the reaction mixture, followed by phase separation. The aqueous layer was removed to obtain the organic layer. The organic layer thus obtained was washed with an aqueous solution of sodium hydroxide (an aqueous solution (210.51 g) of 25% by mass sodium hydroxide and water (526.36 g)), followed by phase separation. The reaction mixture was then washed with an aqueous solution of acetic acid (prepared from acetic acid (41.93 g) and water (526.36 g)), followed by phase separation. The reaction mixture was then washed with an aqueous solution of sodium bicarbonate (prepared from sodium bicarbonate (20.92 g) and water (526.36 g)), followed by phase separation. The organic layer thus obtained was then concentrated at a reduced pressure, and a resulting concentrate was distilled at a reduced pressure to form a 2-halo-6,10-dimethyltridecane compound (1: X¹ = Cl) (444.75 g, 1.64 mol, purity 91.25%, b.p. = 107.1 to 119.0°C/0.044 kPa (0.33 mmHg)) with a yield of 82.18%. An elimination reaction proceeded in the reaction, forming alkene compounds such as 6,10-dimethyl-1-tridecene with a total yield of 8.0%. The 2-halo-6,1 0-dimethyltridecane compound (1: X¹ = Cl) after distillation contained 6.5% of the aforesaid alkenes.

The following is the spectrum data of the 2-chloro-6,10-dimethyltridecane (1: X¹ = Cl) thus obtained.

Nuclear magnetic resonance spectrum: ¹H-NMR (500 MHz, CDCl₃): δ = 0.82-0.91 (9H, m), 1.02-1.56 (19H, m), 1.60-1.76 (2H, m), 4.03 (1H, tq, J = 6.5 Hz, 6.5 Hz); ¹³C-NMR (125 MHz, CDCl₃): δ = 14.40, 19.55, 19.63, 19.67, 19.69, 20.14, 24.14, 24.17, 24.40, 24.42, 25.33, 25.39, 32.47, 32.49, 32.63, 32.66, 32.68, 36.40, 36.46, 36.48, 36.55, 37.23, 37.27, 37.31, 37.34, 37.37, 37.39, 39.37, 39.47, 40.66, 40.71, 58.93.

Mass spectrum: EI-mass spectrum (70 eV): m/z 210 (M⁺-36), 195, 182, 167, 154, 133, 111, 97, 85, 71, 55, 43, 29.

Infrared absorption spectrum: (D-ATR): v = 2956, 2927, 1462, 1378, 740, 616.

### Example 4: Preparation of methyl 2,6,10-trimethyltridecanoate (11: R² = CH₃)

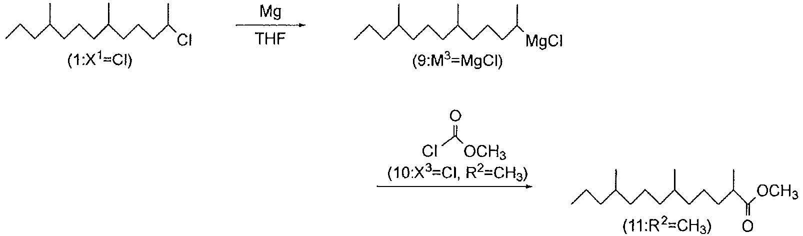

Magnesium (40.08 g, 1.65 gram atoms) and tetrahydrofuran (471.30 g) were placed in a reactor at a room temperature and then stirred for 32 minutes at 60 to 65°C. 2-Chloro-6,10-dimethyltridecane (1: X¹ = Cl) (425.00 g, 1.57 mol, purity 91.25%) prepared as in Example 3 was then added dropwise to the reactor at 60 to 75°C. After the completion of the dropwise addition, the reaction mixture was stirred for 2 hours at 75 to 80°C to form 1,5,9-trimethyldodecylmagnesium chloride (9: M³ = MgCl).

Methyl chloroformate (178.13 g, 1.89 mol, purity 100%) and tetrahydrofuran (314.20 g) were then placed in another reactor different from the aforesaid reactor. The internal temperature of the other reactor was cooled to 0 to 10°C, and then the aforesaid 1,5,9-trimethyldodecylmagnesium chloride (9: M³ = MgCl) was added dropwise to the other reactor at an internal temperature of 0 to 10°C. After the completion of the dropwise addition, the reaction mixture was stirred for 3 hours at 0 to 10°C. An aqueous solution of acetic acid (prepared from acetic acid (9.57 g) and water (538.63 g)) was then added to the reaction mixture, followed by phase separation. The organic layer thus obtained was then concentrated at a reduced pressure, and the resulting concentrate was distilled at a reduced pressure to form methyl 2,6,10-trimethyltridecanoate (11: R² = CH₃) (335.18 g, 1.21 mol, purity 97.96%, b.p. = 140.1 to 149.0°C/0.045 kPa (0.34 mmHg)) with a yield of 77.26%. Alkenes such as 6,10-dimethyl-1-tridecene that could not be separated in a previous step were completely separated by distillation at this time, and were not present in methyl 2,6,10-trimethyltridecanoate after distillation.

The following is the spectrum data of the methyl 2,6,10-trimethyltridecanoate (11: R² = CH₃) thus obtained.

Nuclear magnetic resonance spectrum: ¹H-NMR (500 MHz, CDCl₃): δ =; 0.83 (6H, d-like, J = 6.5 Hz), 0.87 (3H, t, J = 6.9 Hz), 1.00-1.67 (21H, m), 2.43 (1H, tq, J = 6.9 Hz, 6.9 Hz), 3.66 (3H, s)¹³C-NMR (125 MHz, CDCl₃): δ = 14.38, 16.99, 17.10, 19.56, 19.61, 19.67, 20.11, 24.41, 24.64, 24.66, 24.69, 32.46, 32.48, 32.58, 32.62, 34.09, 34.13, 36.81, 36.90, 37.28, 37.31, 37.33, 37.36, 37.39, 39.37, 39.44, 39.46, 51.40, 177.39.

Mass spectrum: EI-mass spectrum (70 eV): m/z 270 (M⁺), 255, 241, 227, 213, 199, 180, 157, 143, 129, 111, 88, 43, 29.

Infrared absorption spectrum: (D-ATR): ν = 2955, 2928, 1741, 1463, 1378, 1195, 1167, 740.

### Example 5: Preparation of hexyl 2,6,10-trimethyltridecanoate (11: R² = (CH₂)₅CH₃)

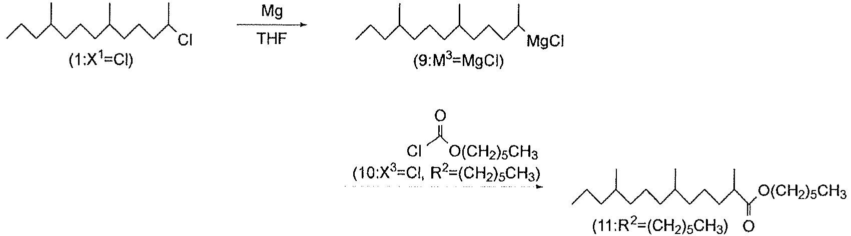

Magnesium (3.25 g, 0.13 gram atoms) and tetrahydrofuran (38.26 g) were placed in a reactor at a room temperature and then stirred for 18 minutes at 60 to 65°C. 2-Chloro-6,10-dimethyltridecane (1: X¹ = Cl) (34.24 g, 0.13 mol, purity 91.92%) prepared as in Example 3 was then added dropwise to the reactor at 60 to 75°C. After the completion of the dropwise addition, the reaction mixture was stirred for 2 hours at 75 to 80°C to form 1,5,9-trimethyldodecylmagnesium chloride (9: M³ = MgCl).

Hexyl chloroformate (25.19 g, 0.15 mol, purity 100%) and tetrahydrofuran (25.50 g) were then placed in another reactor different from the aforesaid reactor, and the internal temperature of the other reactor was cooled to 0 to 10°C. The aforesaid 1,5,9-trimethyldodecylmagnesium chloride (9: M³ = MgCl) was then added dropwise to the other reactor at an internal temperature of 0 to 10°C. After the completion of the dropwise addition, the reaction mixture was stirred for 2.5 hours at 0 to 10°C. An aqueous solution of acetic acid (prepared from acetic acid (0.78 g) and water (43.71 g)) was then added to the reaction mixture, followed by phase separation. The organic layer thus obtained was then concentrated at a reduced pressure, and the resulting concentrate was purified by silica gel column chromatography (hexane:ethyl acetate = 50:1 to 20:1) to form hexyl 2,6,10-trimethyltridecanoate (11: R² = (CH₂)₅CH₃) (39.41 g, 0.11 mol, purity 97.48%) with a yield of 88.44%.

The following is the spectrum data of the hexyl 2,6,10-trimethyltridecanoate (11: R² = (CH₂)₅CH₃) thus obtained.

Nuclear magnetic resonance spectrum: ¹H-NMR (500 MHz, CDCl₃): δ = 0.83 (6H, d-like, J = 6.5 Hz), 0.88 (6H, q-like, J = 6.9 Hz), 1.01-1.11 (4H, m), 1.13 (3H, d, J = 6.9 Hz), 1.18-1.42 (19H, m), 1.58-1.68 (3H, quin-like, J = 6.9 Hz), 2.42 (1H, tq, J = 6.9 Hz), 4.01-4.10 (2H, m); ¹³C-NMR (125 MHz, CDCl₃): δ = 13.98, 14.39, 17.08, 17.18, 19.57, 19.61, 19.63, 19.67, 20.12, 22.53, 24.42, 24.67, 24.70, 25.60, 28.64, 31.43, 32.47, 32.49, 32.63, 32.65, 32.67, 34.15, 34.18, 36.86, 36.96, 37.32, 37.35, 37.40, 39.37, 39.47, 39.63, 39.66, 64.27, 177.02.

Mass spectrum: EI-mass spectrum (70 eV): m/z 340 (M⁺), 257, 227, 199, 180, 158, 129, 113, 97, 74, 43.

Infrared absorption spectrum: (D-ATR): v = 2956, 2929, 1737, 1463, 1378, 1259, 1169.

### Example 6: Preparation of decyl 2,6,10-trimethyltridecanoate (11: R² = (CH₂)₉CH₃)

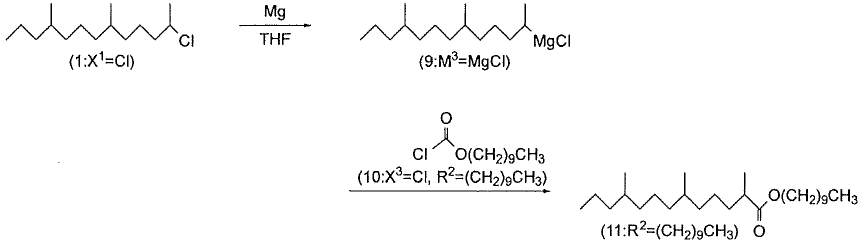

Magnesium (2.31 g, 0.095 gram atoms) and tetrahydrofuran (27.18 g) were placed in a reactor at a room temperature and then stirred for 18 minutes at 60 to 65°C. 2-Chloro-6,10-dimethyltridecane (1: X¹ = Cl) (24.33 g, 0.091 mol, purity 91.92%) prepared as in Example 3 was then added dropwise to the reactor at 60 to 75°C. After the completion of the dropwise addition, the reaction mixture was stirred for 2 hours at 75 to 80°C to form 1,5,9-trimethyldodecylmagnesium chloride (9: M³ = MgCl).

Decyl chloroformate (24.00 g, 0.11 mol, purity 100%) and tetrahydrofuran (18.12 g) were then placed in another reactor different from the aforesaid reactor, and the internal temperature of the other reactor was cooled to 0 to 10°C. The aforesaid 1,5,9-trimethyldodecylmagnesium chloride (9: M³ = MgCl) was then added dropwise to the other reactor at an internal temperature of 0 to 10°C. After the completion of the dropwise addition, the reaction mixture was stirred for 2.5 hours at 0 to 10°C. An aqueous solution of acetic acid (prepared from acetic acid (0.55 g) and water (31.06 g)) was then added to the reaction mixture, followed by phase separation. The organic layer thus obtained was then concentrated at a reduced pressure, and the resulting concentrate was purified by silica gel column chromatography (hexane:ethyl acetate = 50:1 to 20:1) to form decyl 2,6,10-trimethyltridecanoate (11: R² = (CH₂)₉CH₃) (31.28 g, 0.078 mol, purity 98.41%) with a yield of 85.66%.

The following is the spectrum data of the decyl 2,6,10-trimethyltridecanoate (11: R² = (CH₂)₉CH₃) thus obtained.

Nuclear magnetic resonance spectrum: ¹H-NMR (500 MHz, CDCl₃): δ = 0.83 (6H, d-like, J = 6.9 Hz), 0.87 (6H, td, J = 7.1 Hz, 2.7 Hz), 1.01-1.11 (4H, m), 1.13 (3H, d, J = 6.9 Hz), 1.19-1.41 (26H, m), 1.57-1.68 (3H, m), 2.42 (2H, tq, J = 6.9 Hz, 6.9 Hz), 4.01-4.11 (2H, m); ¹³C-NMR (125 MHz, CDCl₃): δ = 14.09, 14.40, 17.08, 17.18, 19.58, 19.61, 19.64, 19.67, 20.12, 20.14, 22.67, 24.43, 24.69, 25.94, 28.68, 29.25, 29.30, 29.53, 31.88, 32.48, 32.49, 32.63, 32.65, 32.67, 34.16, 34.18, 36.87, 36.96, 37.31, 37.33, 37.36, 37.38, 37.41, 39.38, 39.48, 39.64, 39.66, 64.28, 177.03.

Mass spectrum: EI-mass spectrum (70 eV): m/z 396 (M⁺), 325, 283, 257, 214, 180, 140, 115, 97, 74, 43.

Infrared absorption spectrum: (D-ATR): v = 2956, 2926, 1737, 1463, 1378, 1168.

## Claims

1. A 2-halo-6,10-dimethyltridecane compound of the following general formula (1): 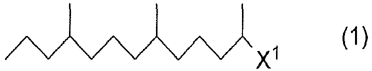 wherein X¹ represents a halogen atom.

2. A process for preparing a 2-halo-6,10-dimethyltridecane compound of the following general formula (1): 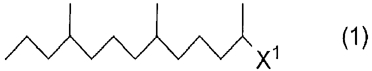
wherein X¹ represents a halogen atom,
the process comprising steps of:
subjecting a nucleophilic reagent, 3,7-dimethyldecyl compound, of the following general formula (2): 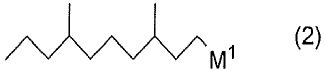
wherein M¹ represents Li, MgZ¹, CuZ¹, or CuLiZ¹, and Z¹ represents a halogen atom or a 3,7-dimethyldecyl group,
to a nucleophilic addition reaction with propylene oxide of the following formula (3): 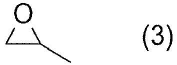
to form 6,10-dimethyl-2-tridecanol of the following formula (4): 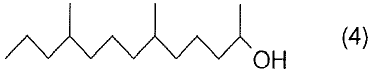 and
reacting the 6,10-dimethyl-2-tridecanol (4) thus obtained with a halogenating agent (5) to form the aforesaid 2-halo-6,10-dimethyltridecane compound (1).

3. The aforesaid process for preparing the 2-halo-6,10-dimethyltridecane compound (1) according to claim 2, the process further comprising steps of:
subjecting a nucleophilic reagent, 2-methylpentyl compound, of the following general formula (6): 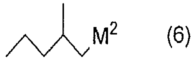
wherein M² represents Li, MgZ², CuZ², or CuLiZ², and Z² represents a halogen atom or a 2-methylpentyl group,
to a coupling reaction with a sulfonate compound (7) of the following general formula (7): 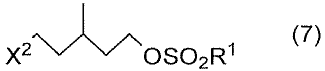
wherein X² represents a halogen atom, and R¹ represents a hydrocarbon group having 1 to 7 carbon atoms,
to form a 1-halo-3,7-dimethyldecane compound of the following general formula (8): 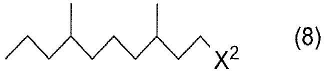
wherein X² represents a halogen atom,
and
converting the 1-halo-3,7-dimethyldecane compound (8) thus obtained into the aforesaid nucleophilic reagent, 3,7-dimethyldecyl compound (2).

4. A process for preparing an alkyl 2,6,10-trimethyltridecanoate compound of the following general formula (11): 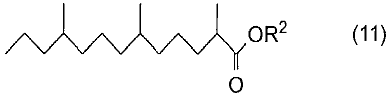
wherein R² represents an alkyl group having 1 to 12 carbon atoms,
the process comprising steps of:
converting a 2-halo-6,10-dimethyltridecane compound of the following general formula (1): 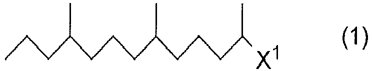
wherein X¹ represents a halogen atom,
into a nucleophilic reagent, 1,5,9-trimethyldodecyl compound, of the following general formula (9): 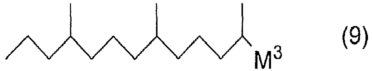 and
subjecting the nucleophilic reagent, 1,5,9-trimethyldodecyl compound (9) thus obtained, to a nucleophilic substitution reaction with alkyl haloformate of the following general formula (10): 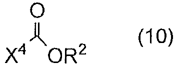
wherein X⁴ represents a halogen atom, and R² represents an alkyl group having 1 to 12 carbon atoms,
to form the aforesaid alkyl 2,6,10-trimethyltridecanoate compound (11).
